# EUROPEAN PATENT SPECIFICATION

(11) **EP 2 891 494 B1**
(45) Date of publication and mention of the grant of the patent: **19.07.2023**
(21) Application number: 13832739.0
(22) Date of filing: 30.08.2013
(51) Int. Cl.: A61K 31/5517, A61K 31/4468, A61K 31/567, A61K 45/00, A61P 25/20

(54) **METHOD FOR ADMINISTERING HYPNOTIC/SEDATIVE AGENT**
VERFAHREN ZUR VERABREICHUNG VON HYPNOSE-/BERUHIGUNGSMITTELN
MÉTHODE D'ADMINISTRATION D'UN AGENT HYPNOTIQUE/SÉDATIF

(30) Priority: 31.08.2012 JP 2012192081
(43) Date of publication of application: 08.07.2015
(73) Proprietor: PAION UK Limited, Richmond, TW9 2 PR London (GB)
(72) Inventor: KONDO, Maki, Osaka-shi Osaka 541-8564 (JP); KONOMI, Toshihiko, Osaka-shi Osaka 541-8564 (JP); SATO, Shigehito, Hamamatsu-shi Shizuoka 431-3192 (JP); DOI, Matsuyuki, Hamamatsu-shi Shizuoka 431-3192 (JP)
(74) Representative: König Szynka Tilmann von Renesse Patentanwälte Partnerschaft mbB Düsseldorf
(86) International application number: PCT/JP2013/073414
(87) International publication number: WO 2014/034890

(56) References cited:
- WO-A1-2012/062439
- JP-A- 2002 544 266
- JP-A- 2009 542 785
- ANTONIK, LJ. ET AL.: 'A placebo- and Midazolam- controlled phase I single ascending-dose study evaluating the safety, pharmacokinetics, and pharmacodynamics of Remimazolam(CNS 7056):Part I. Safety, efficacy, and basic pharmacokinetics' ANESTHESIA & ANALGESIA vol. 115, no. 2, August 2012, pages 274 - 283, XP055234764
- UPTON, RN. ET AL.: 'A dose escalation study in sheep of the effects of the benzodiazepine CNS 7056 on sedation, the EEG and the respiratory and cardiovascular systems' BRITISH JOURNAL OF PHARMACOLOGY vol. 155, 2008, pages 52 - 61, XP055039848
- UPTON, RN. ET AL.: 'Pharmacokinetics and pharmacodynamics of the short-acting sedative CNS 7056 in sheep' BRITISH JOURNAL OF ANAESTHESIA vol. 105, no. 6, 2010, pages 798 - 809, XP009154068
- WILTSHIRE, HR. ET AL.: 'A placebo- and Midazolam-controlled phase I single ascending- dose study evaluating the safety, pharmacokinetics, and pharmacodynamics of Remimazolam(CNS 7056):Part II. Population Pharmacokinetic and Pharmacodynamic Modeling and Simulation' ANESTHESIA & ANALGESIA vol. 115, no. 2, August 2012, pages 284 - 296, XP055234768

## Description

### [Technical Field]

The present invention relates to a dosing regimen of a sedative comprising, as an active ingredient, methyl 3-[(4S)-8-bromo-1-methyl-6-(2-pyridinyl)-4H-imidazo[1,2-a] [1,4]benzodiazepin-4-yl]propanoate, which is a benzodiazepine compound, or a salt thereof. More particularly, the invention relates to a dosing regimen of the sedative most suitable for the induction and maintenance of general anesthesia.

### [Background Art]

General anesthesia is widely used for the purpose of eliminating physical and mental pain of a patient caused by surgery. Requirements necessary for general anesthesia are as follows: to cause loss of consciousness in a patient (sedation); to eliminate pain (analgesia); to cause loss of body motor (muscle relaxation); and to get rid of unwanted reflexes such as throat seizure and arrhythmia (reflex inhibition). However, it is difficult to satisfy all of the requirements using a single anesthetic. Further, the degree of necessity of the requirements for general anesthesia varies depending on the surgery to be performed, and therefore, when general anesthesia is used, in order to satisfy the respective requirements for general anesthesia, a sedative, an analgesic, a muscle relaxant, etc. are administered while adjusting the doses thereof according to the type of surgery and the individual patient. Such a technique is called "balanced anesthesia" and has been regarded as a highly valuable technique for achieving ideal anesthesia while minimizing side effects of respective agents by combining various agents.

Sedatives which are used in general anesthesia to cause loss of consciousness in a patient and maintain the state of loss of consciousness are roughly divided into an intravenous anesthetic and an inhalational anesthetic. The intravenous anesthetic has advantages not shared by an inhalational anesthetic, for example, the intravenous anesthetic can be used with a relatively simple device, there is no stage of excitement at the time of induction of general anesthesia, contamination in the surgery room or air is not caused, etc. Therefore, since a product containing propofol was placed on the market in 1995, the intravenous anesthetic has spread rapidly in Japan. Further, in 2007, a product containing remifentanil hydrochloride, which is a short-acting narcotic analgesic and can be intravenously administered, and a product containing rocuronium bromide, which is a muscle relaxant and can be intravenously administered, were placed on the market in succession, and a total intravenous anesthesia (TIVA) technique which satisfies all of the requirements necessary for general anesthesia using agents which can be continuously and intravenously administered is gradually spreading.

As the intravenous anesthetic for the purpose of sedation, a product containing, as an active ingredient, propofol, midazolam, thiamylal sodium, or the like has been often used.

The duration of sedative effect of propofol is short, and even if propofol is administered for a long time, the time from completion of administration to recovery from anesthesia is short, and therefore, propofol is used for the induction and maintenance of general anesthesia. However, propofol has been known to have problems that circulatory depression such as hypotension, occurrence of angialgia, risk of microbial contamination, and intraoperative awakening are likely to be caused, a burning sensation is caused due to the activation of TRPA1 channel, etc. Further, it has been noted that propofol has a risk of causing propofol infusion syndrome, although the occurrence is rare. The propofol infusion syndrome is a disease caused in a patient to whom propofol has been administered for long-term sedation in an intensive care unit and is a fatal complication in which metabolic acidosis, dyslipidemia, and multiple organ failure progress to cause bradyarrhythmia and lead to cardiac arrest.

On the other hand, midazolam, which is a benzodiazepine hypnotic, has advantages that the circulatory depressive effect is lower than propofol, it has an amnesic effect useful for the prevention of a posttraumatic stress disorder due to intraoperative awakening, there is an antagonist therefor, etc. However, the duration of sedative effect of midazolam is long, and therefore, it is necessary to use midazolam by intermittent administration not by continuous administration, and midazolam is not so suitable for practical use in the induction and maintenance of general anesthesia. In addition, if midazolam is administered for a long time, recovery from anesthesia after stopping the administration is delayed, and therefore, a time until a patient can leave the surgery room is prolonged to increase the burden on the patient and also the medical staff is tied down for a long time.

Further, thiamylal sodium exhibits a sedative effect rapidly at the time of induction of general anesthesia and a time until recovery from anesthesia after stopping the administration is short, and therefore thiamylal sodium is used for the induction of general anesthesia. However, similarly to midazolam, if thiamylal sodium is administered for a long time, recovery from anesthesia after stopping the administration is delayed, and therefore, thiamylal sodium is not used for the purpose of maintenance of general anesthesia.

As described above, intravenous anesthetics for the purpose of sedation currently used in a clinical site have advantages and disadvantages, respectively, and the development of a novel intravenous anesthetic having only the respective advantages has been demanded.

On the other hand, methyl 3-[(4S)-8-bromo-1-methyl-6- (2-pyridinyl) -4H-imidazo [1, 2-a] [1, 4] benzodiazepin-4-yl]propanoate is a benzodiazepine compound disclosed in WO 2000/069836, and it is described that the compound is a short-acting central nervous system depressant that is useful for intravenous administration in the following clinical settings: preoperative sedation, anxiolysis, and amnestic use for perioperative events; conscious sedation during short diagnostic, operative or endoscopic procedures; as a component for the induction and maintenance of general anesthesia prior and/or concomitant to the administration of other anesthetics; and ICU sedation. Further, it is described that this compound may form a pharmaceutically acceptable salt with benzenesulfonic acid or the like, and by intravenously administering such a compound through bolus injection, preferably continuous infusion to a mammal at a dose of from 0.01 to 5.0 mg/kg of body weight, and preferably from 0.02 to 0.5 mg/kg of body weight, a sedative or hypnotic effect can be obtained (see Patent Document 1).

Further, for example, in WO 2008/007071, a benzenesulfonic acid salt of methyl 3-[(4S)-8-bromo-1-methyl-6-(2-pyridinyl)-4H-imidazo[1,2-a] [1,4]benzodiazepin-4-yl]propanoate and a crystalline polymorphism thereof are disclosed (see Patent Document 2) .

Further, for example, in WO 2012/062439, a method for obtaining a sedative effect by administering methyl 3-[(4S)-8-bromo-l-methyl-6-(2-pyridinyl)-4H-imidazo[1,2-a][1,4]benzodiazepin-4-yl]propanoate, a salt thereof, or a solvate thereof in combination with an opioid at a dose of from 2 to 10 mg is disclosed (see Patent Document 3).

Still further, for example, in Anesthesia and Analgesia, vol. 115, pp. 274-283, 2012 and Anesthesia and Analgesia, vol. 115, pp. 284-296, 2012, the evaluation results obtained by comparing safety, tolerability, pharmacokinetics, pharmacological effects, etc. between a placebo and midazolam when performing single intravenous administration of remimazolam (methyl 3-[(4S)-8-bromo-1-methyl-6-(2-pyridinyl)-4H-imidazo[1,2-a] [1,4]benzodiazepin-4-yl]propanoate) over 1 minute in a double-blind manner are described (see Non-Patent Documents 1 and 2).

Further, for example, in British Journal of Pharmacology, vol. 155, pp. 52-61, 2008 and British Journal of Anaesthesia, vol. 105, No. 6, pp. 798-809, 2010, it is described that continuous intravenous injection to sheep was performed for 2 minutes using CNS 7056 (methyl 3-[(4S)-8-bromo-1-methyl-6-(2-pyridinyl)-4H-imidazo[1,2-a][1,4]benzodiazepin-4-yl]propanoate) (see Non-Patent Documents 3 and 4).

As described above, when general anesthesia is introduced, a plurality of agents are simultaneously applied to a patient, and it is by no means rare that by combining a plurality of agents, an adverse effect is exhibited in a patient or a desired drug efficacy cannot be obtained. Also, it is not difficult to predict that a possibility of the onset of toxicity is increased by performing the long-term continuous administration of a compound even if the compound has been confirmed to be safe in single administration or short-term continuous administration.

For example, it is known that sensitivity to a benzodiazepine compound is increased in the elderly and also it is said that the sensitivity varies depending on the gender. It is true in the case of a narcotic analgesic. For example, it is said that the clearance of remifentanil is decreased by 25% in patients at the age of 65 or more, and also that a time until the concentration of remifentanil in plasma reaches equilibrium with that in the site of action is delayed. Further, in general, when a benzodiazepine compound is used in combination with a narcotic analgesic, a synergistic effect is obtained, and therefore, it is considered that when these compounds are used in combination, the respective doses should be decreased.

In the case where a novel dosing regimen such as long-term continuous administration is to be employed by using a plurality of agents in combination, it is not sufficient that safety in the single administration of each agent has been confirmed, and a method capable of obtaining a safer and more adequate drug efficacy should be selected in consideration of the interaction by the combination of such agents, the toxicity and accumulation of the compounds or metabolites thereof by long-term continuous administration, etc.

However, in the case where a sedative comprising methyl 3-[(4S)-8-bromo-1-methyl-6-(2-pyridinyl)-4H-imidazo[1,2-a] [1,4]benzodiazepin-4-yl]propanoate or a salt thereof is to be administered to a patient for the purpose of the induction and/or maintenance of general anesthesia, since such a compound has been continuously administered to sheep for only 2 minutes and to a human for only 1 minute, specifically what dosing regimen should be used for the compound was totally unknown so as to be able to use the compound as a safe and useful general anesthetic having the advantages of propofol (for example, the adjustability is high, and a recovery time from anesthesia after stopping the administration is short) and the advantages of midazolam, which is a benzodiazepine hypnotic (for example, the activity of causing circulatory depression such as hypotension is low, and there is an antagonist for midazolam) in the case where the compound is used in combination with other concomitant agents as well as in the case where the compound is used alone.

### [Prior Art Documents]

### [Patent Documents]

[Patent Document 1] WO 2000/069836
[Patent Document 2] WO 2008/007071
[Patent Document 3] WO 2012/062439

### [Non-Patent Documents]

[Non-Patent Document 1] Anesthesia and Analgesia, vol. 115, pp. 274-283, 2012
[Non-Patent Document 2] Anesthesia and Analgesia, vol. 115, pp. 284-296, 2012
[Non-Patent Document 3] British Journal of Pharmacology, vol. 155, pp. 52-61, 2008
[Non-Patent Document 4] British Journal of Anaesthesia, vol. 105, No. 6, pp. 798-809, 2010

### [Summary of the Invention]

### [Problems that the Invention is to Solve]

An object of the invention is to provide a most suitable dosing regimen of a sedative comprising, as an active ingredient, methyl 3-[(4S)-8-bromo-1-methyl-6-(2-pyridinyl)-4H-imidazo[1,2-a] [1,4]benzodiazepin-4-yl]propanoate or a salt thereof when it is used for the induction and maintenance of general anesthesia.

### [Means for Solving the Problems]

The present inventors made intensive studies in order to achieve the above object, and as a result, they found that the below-described specific dosing regimen is most suitable when a sedative comprising, as an active ingredient, methyl 3-[(4S)-8-bromo-1-methyl-6-(2-pyridinyl) -4H-imidazo [1, 2-a] [1, 4] benzodiazepin-4-yl]propanoate or a salt thereof is used for the induction and maintenance of general anesthesia, and then, further made intensive studies, thereby completing the invention.

That is, the present invention relates to a compound for use in sedation as specified in the claims, wherein the compound is methyl 3-[(4S)-8-bromo-1-methyl-6-(2-pyridinyl)-4H-imidazo[1,2-a] [1,4]benzodiazepin-4-yl]propanoate or a salt thereof, and further to a compound for use in inducing loss of consciousness as specified in the claims, wherein the compound is methyl 3-[(4S)-8-bromo-1-methyl-6-(2-pyridinyl)-4H-imidazo[1,2-a][1,4]benzodiazepin-4-yl]propanoate benzenesulfonate.

### [Advantage of the Invention]

According to the invention, a dosing regimen of a sedative comprising methyl 3-[(4S)-8-bromo-1-methyl-6-(2-pyridinyl)-4H-imidazo[1,2-a][1,4]benzodiazepin-4-yl]propanoate, which is a benzodiazepine compound, or a salt thereof is disclosed. The dosing regimen is most suitable when using the benzodiazepine compound for the induction and maintenance of general anesthesia and employs intravenous administration, and therefore is particularly useful for total intravenous anesthesia (TIVA). By using the dosing regimen of the invention, rapid loss of consciousness is caused to place a patient in a state of general anesthesia after the administration is started and also rapid recovery of consciousness occurs after stopping the administration and recovery from the state of general anesthesia can be achieved. In addition, a side effect such as hypotension is mild and an adequate depth of anesthesia can be maintained, and therefore, intraoperative awakening does not occur. Even if intraoperative awakening occurs, the benzodiazepine compound is expected to exhibit an amnesic effect based on the benzodiazepine skeleton of the compound so as not to cause a posttraumatic stress disorder due to the memory upon awakening during surgery.

### [Mode for Carrying Out the Invention]

In the invention, methyl 3-[(4S)-8-bromo-1-methyl-6-(2-pyridinyl)-4H-imidazo[1,2-a][1,4]benzodiazepin-4-yl]propanoate (hereinafter sometimes abbreviated as "Compound A") is a known compound represented by the following formula (A), and is described in, for example, Example Ic-8 in WO 2000/069836. The compound is sometimes also referred to as "remimazolam" or "CNS 7056".

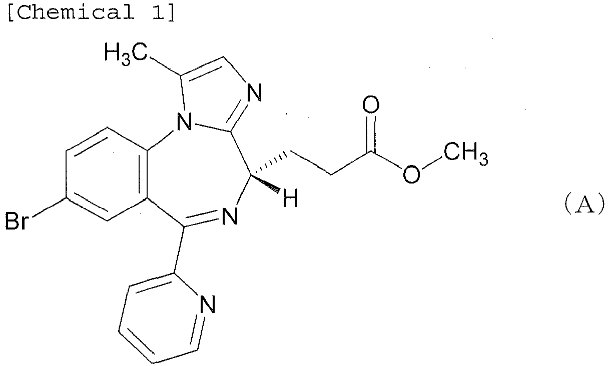

In the invention, unless otherwise specified, as apparent to those skilled in the art, the symbol 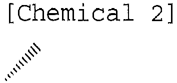 indicates a bond going behind the plane of the paper (that is, α-configuration); the symbol 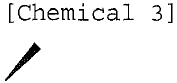 indicates a bond coming out of the plane of the paper (that is, β-configuration); and the symbol 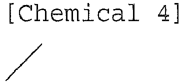 indicates an α-configuration, β-configuration, or a mixture thereof at an arbitrary ratio.

In the invention, examples of the salt of methyl 3-[(4S)-8-bromo-1-methyl-6-(2-pyridinyl)-4H-imidazo[1,2-a] [1,4]benzodiazepin-4-yl]propanoate include salts described in WO 2000/069836 such as hydrochlorides, hydrobromides, sulfates, nitrates, phosphates, salicylates, p-toluenesulfonates, tartarates, citrates, methanesulfonates, maleates, formates, malonates, succinates, isethionates, lactobionates, naphthalene-2-sulfonates, sulfamates, ethanesulfonates, and benzenesulfonates.

In the invention, as one of the preferred embodiments of Compound A and a salt thereof, methyl 3-[(4S)-8-bromo-1-methyl-6-(2-pyridinyl)-4H-imidazo[1,2-a] [1,4]benzodiazepin-4-yl]propanoate benzenesulfonate can be exemplified. Methyl 3-[(4S)-8-bromo-1-methyl-6-(2-pyridinyl)-4H-imidazo[1,2-a][1,4]benzodiazepin-4-yl]propanoate benzenesulfonate (hereinafter sometimes abbreviated as "Compound B") is a known compound represented by the following formula (B), and is described in, for example, WO 2008/007071 as a besilate.

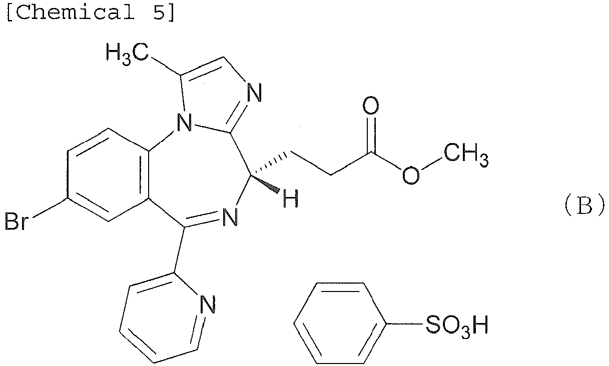

In the invention, Compound A or a salt thereof and Compound B can be produced by appropriately combining known methods, for example, methods described in WO 2000/069836, WO 2008/007071, WO 2008/007081, WO 2011/032692, and WO 2012/062439, or similar methods thereto, etc. In addition, such a compound can also be produced by using general methods other than the above methods, for examples, methods described in Comprehensive Organic Transformations: A Guide to Functional Group Preparations, 2nd Edition (Richard C. Larock, John Wiley & Sons, Inc., 1999), etc., or partially modified methods thereof, etc. in an appropriate combination.

Compound A or a salt thereof and Compound B are grouped in a benzodiazepine compound based on the structure thereof. Further, midazolam having the following structure is also grouped in a benzodiazepine compound.

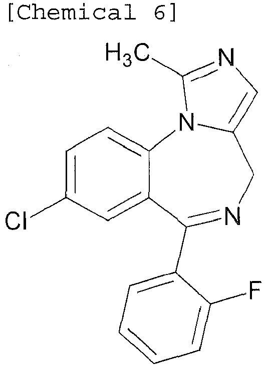

The benzodiazepine compound is a collective term of a group of compounds having a partial structure of a bicyclic heterocycle in which a benzene ring and a 7-membered ring having 2 nitrogen atoms are condensed in its structural formula. Many of the benzodiazepine compounds have an activity to induce hypnosis by activating the GABA_{A} receptor to promote the inflow of chloride ions, and therefore are used as an active ingredient of a sedative (a benzodiazepine sedative) as a benzodiazepine sedative agent.

In the invention, the sedative comprising Compound A or a salt thereof as an active ingredient refers to a pharmaceutical composition comprising Compound A or a salt thereof as an active ingredient for the induction of a sedative and/or sedative state in a mammal (preferably a human, more preferably a patient) which (who) receives the administration.

Such a pharmaceutical composition may be any as long as it is a preparation obtained by formulation of Compound A or a salt thereof as an active ingredient along with a variety of pharmaceutically acceptable carriers such as additives and solvents, however, a composition for intravenous administration is preferably used. Here, the pharmaceutically acceptable carrier refers to a substance other than the active ingredient to be generally used in a pharmaceutical preparation. As the pharmaceutically acceptable carrier, a substance which does not exhibit a pharmacological effect at a dose of the preparation, is harmless, and does not inhibit the pharmacological effect of the active ingredient is preferred. Further, the pharmaceutically acceptable carrier can be used also for the purpose of enhancing the usefulness of the active ingredient and the preparation, facilitating the formulation, stabilizing the quality, improving the usability, etc. Specifically, a substance described in "Japanese pharmaceutical excipients" (edited by The Japan Pharmaceutical Excipients Council) published by YAKUJI NIPPO LIMITED in 2000 may be appropriately selected according to need.

The pharmaceutical composition for intravenous administration may be in the form of a solution, a suspension, an emulsion, or a solid sterile preparation (such as a lyophilized injection) which is used by dissolving or suspending immediately before use, and can be generally produced by the following method (a), (b) or (c) :
(a) a method in which the active ingredient as such or a mixture obtained by adding an additive to the active ingredient is dissolved, suspended, or emulsified in an water for injection or another aqueous solvent or a nonaqueous solvent, etc. and then homogenized, and the resulting product is filled into a container for injection and the container is sealed, followed by sterilization;
(b) a method in which the active ingredient as such or a mixture obtained by adding an additive to the active ingredient is dissolved, suspended, or emulsified in an water for injection or another aqueous solvent or a nonaqueous solvent, etc. and then homogenized, followed by sterile filtration, or preparation is aseptically performed, followed by homogenization, and the resulting product is filled into a container for injection and the container is sealed; or
(c) a method in which the active ingredient as such or the active ingredient and an additive such as an excipient, etc. is/are dissolved in an water for injection, followed by sterile filtration, and the resulting product is filled into a container for injection and thereafter lyophilized, or the resulting product is lyophilized in a dedicated container and thereafter directly filled into a container.

The invention discloses a dosing regimen for using a sedative comprising Compound A or a salt thereof as an active ingredient for the induction and maintenance of general anesthesia, and further, the invention is characterized by the dosing regimen.

In the invention, the induction of general anesthesia refers to a treatment of placing a patient who is not in a state of general anesthesia in a state of general anesthesia. Specifically, the induction of general anesthesia is performed by administering an agent for inducing general anesthesia in an amount necessary for inducing general anesthesia to a patient. That is, in the invention, the induction of general anesthesia can be performed by administering a sedative comprising Compound A or a salt thereof as an active ingredient in an amount necessary for inducing general anesthesia to a patient.

Whether or not the patient is in a state of general anesthesia can be determined by the presence or absence of consciousness in the patient. Whether or not the patient is conscious after the induction of general anesthesia is started can be determined by, for example, asking a simple question requiring a response of the patient or observing a response to physical stimulation applied to the body of the patient, etc. An example of a simple procedure for determining whether or not the patient is conscious is as follows. For example, a procedure in which the patient is made to count aloud along with the induction of general anesthesia, and after the patient stops counting aloud, the shoulder of the patient is shaken or the like can be exemplified. It can be determined that if the patient does not respond when the shoulder is shaken, the patient is not conscious, if the patient responds, the patient is conscious.

In the invention, the maintenance of general anesthesia refers to a treatment of maintaining the state of general anesthesia in a patient having been placed in a state of general anesthesia. Specifically, the maintenance of general anesthesia can be performed by administering an agent for maintaining general anesthesia in an amount necessary for maintaining general anesthesia to a patient. That is, in the invention, the maintenance of general anesthesia is performed by administering a sedative comprising Compound A or a salt thereof as an active ingredient in an amount necessary for maintaining general anesthesia to a patient.

In the invention, the induction and maintenance of general anesthesia refers to the induction of general anesthesia and the maintenance of general anesthesia are performed in succession. That is, in the invention, the induction and maintenance of general anesthesia can be performed by administering a sedative comprising Compound A or a salt thereof as an active ingredient in an amount necessary for inducing general anesthesia to a patient so that the patient is placed in a state of general anesthesia, and subsequently, administering the sedative in an amount necessary for maintaining general anesthesia to the patient so that the state of general anesthesia is maintained.

Among the dosing regimens of the sedative comprising Compound A or a salt thereof disclosed in the invention, a dosing regimen to be used for the induction and maintenance of general anesthesia will be described in detail below as the dosing regimen of the invention.

The dosing regimen of the invention includes administering the sedative to a patient by a two-step administration process. More specifically, the administration process includes two steps in total, i.e., one step (first step) for the induction of general anesthesia and one step (second step) for the maintenance of general anesthesia. The chemical concentration of the sedative for such an administration process is not particularly limited. It is not always necessary to use the chemical in the same concentration in the first step and the second step. However, taking into consideration convenience or the like in a clinical site, the chemical concentration of the sedative to be used for the induction and maintenance of general anesthesia is preferably 1 mg/mL.

In the dosing regimen of the invention, it is preferred that the sedative comprising Compound A or a salt thereof is administered continuously to a patient for more than 2 minutes in total in the first step and the second step.

In the dosing regimen of the invention, the first step performed for the induction of general anesthesia includes intravenously administering Compound A or a salt thereof, which is the active ingredient, to a patient who needs a treatment with the compound at a dosing rate of about 3 mg to about 40 mg/hour/kg in the patient, which constitutes one step.

The dosing rate in the first step is preferably, for example, about 4 mg to about 30 mg/hour/kg in a patient who needs a treatment with the compound, more preferably, for example, about 4 mg, about 6 mg, about 8 mg, about 12 mg, about 21 mg, or about 30 mg/hour/kg in a patient who needs a treatment with the compound. Particularly, the dosing rate is preferably about 6 mg or about 12 mg/hour/kg in a patient which needs a treatment with the compound.

In particular, in the case where the patient is a non-elderly adult, the dosing rate is preferably, for example, about 6 mg to about 30 mg/hour/kg in a patient who needs a treatment with the compound, and more preferably, for example, about 6 mg, about 12 mg, about 21 mg, or about 30 mg/hour/kg in a patient who needs a treatment with the compound. Above all, the dosing rate is preferably not more than about 12 mg/hour/kg in a patient who needs a treatment with the compound, and therefore the dosing rate is preferably about 6 mg or about 12 mg/hour/kg in a patient who needs a treatment with the compound.

On the other hand, in the case where the patient is an elderly adult, the dosing rate is preferably, for example, about 4 mg to about 12 mg/hour/kg in a patient who needs a treatment with the compound, and more preferably, for example, about 4 mg, about 8 mg, or about 12 mg/hour/kg in a patient who needs a treatment with the compound. Further, within such a range of the dosing rate, the same dosing rate as in a non-elderly adult, namely about 6 mg or about 12 mg/hour/kg in a patient which needs a treatment with the compound, is also preferred.

In the invention, an elderly adult refers to a patient who is 65 years old or older, and a non-elderly adult refers to a patient who is 20 years old or older and younger than 65 years old.

Further, in the invention, the dosing rate of Compound A or a salt thereof is expressed in, for example, "about 12 mg/hour/kg in a patient who needs a treatment with the compound", however, it should be understood that "12 mg" as used herein denotes the amount of "Compound A" in a free form. In the case of using a salt, it should be understood that "12 mg" denotes the amount of "Compound A contained in the salt" and the amount of the salt should be calculated by adding the amount of an acid component constituting the salt (for example, in the case of Compound B, benzenesulfonic acid) to 12 mg. Accordingly, in the invention, in the case of using, as an active ingredient, a "salt of Compound A", in order to avoid ambiguousness, it is to be noted that the workings "as the amount of Compound A" may be properly compensated for the expression of the dosing rate.

In the dosing regimen of the invention, the second step which is performed for the maintenance of general anesthesia includes intravenous administration of Compound A or a salt thereof, which is the active ingredient, to a patient who needs a treatment with the compound at a dosing rate of about 0.3 mg to about 2.5 mg/hour/kg in the patient, preferably about 0.4 mg to about 1 mg/hour/kg in a patient who needs a treatment with the compound, and more preferably about 1 mg/hour/kg in a patient who needs a treatment with the compound, which constitutes one step. The second step is preferably performed for more than 2 minutes.

In the invention, the unit notation can be appropriately changed within a range that conforms to the standard scientific notation. For example, "about 3 mg per hour per kilogram" can be expressed as "about 3 mg/kg/h" or "about 3 mg/kg/hr", and can also be expressed as "about 0.05 mg/kg/min".

In the dosing regimen of the invention, it is preferred that the dosing rate of the active ingredient in the single step is not changed, however, in the step for the maintenance of general anesthesia (second step), the dosing rate can be appropriately increased or decreased within a range of about 0.3 mg to about 2.5 mg/hour/kg in a patient who needs a treatment with the compound, and preferably within a range of about 2 mg at maximum in a patient who needs a treatment with the compound by using indexes of the general conditions of a patient, for example, signs of awakening of a patient or a BIS value in a patient, as an index. Specifically, for example, in the case of a non-elderly adult, the dosing rate can be appropriately increased or decreased within a range of about 0.4 mg to about 2 mg/hour/kg, specifically within a range of about 0.8 mg to about 2 mg/hour/kg or about 0.4 mg to about 1 mg/hour/kg, in a patient who needs a treatment with the compound, and for example, in the case of an elderly adult, the dosing rate can be appropriately increased or decreased within a range of about 0.4 mg to about 1 mg/hour/kg in a patient who needs a treatment with the compound.

Specifically, in the case where, for example, signs of awakening such as body motion, change in blood pressure or pulse rate, lacrimation, or sweating are observed or a BIS value exceeds 53 in a patient in a state of maintenance of general anesthesia, the dosing rate of Compound A or a salt thereof, which is an active ingredient, can be increased within the above range, and in the case where it is confirmed that a BIS value is 53 or less and signs of awakening are not observed, the dosing rate of Compound A or a salt thereof, which is an active ingredient, can be decreased within the above range.

The BIS as used herein stands for bispectral index, and refers to a parameter obtained by the numerical conversion of an electroencephalographic signal acquired by a sensor attached to the forehead using a calculation processing method developed by Aspect Medical Systems, Inc., and is used for monitoring the depth of anesthesia. The range of the BIS value is from 0 to 100, and a BIS value of 100 denotes an awakening state, and as the depth of anesthesia is increased, the BIS value is decreased. During surgery using general anesthesia, the depth of anesthesia which gives a BIS value between 40 and 60 is considered to be appropriate.

In the case where a sedative comprising Compound A or a salt thereof as an active ingredient is used for the induction and maintenance of general anesthesia in the dosing regimen of the invention, it is preferred to use the sedative in combination with an analgesic such as a narcotic analgesic or a local analgesic and if necessary, further with a muscle relaxant. It is more preferred to use the sedative in combination with a narcotic analgesic in the first step for the induction of general anesthesia, and in combination with a narcotic analgesic and a muscle relaxant in the second step for the maintenance of general anesthesia. The narcotic analgesics to be used in the first step and the second step may not necessarily be the same, and also the administration routes thereof may not necessarily be the same.

In the invention, as the narcotic analgesic, a natural analgesic, a synthetic analgesic, or a semisynthetic analgesic may be used as long as it is an analgesic which has been academically demonstrated to bind to an opioid receptor. However, a narcotic analgesic which exhibits an analgesic effect by intravenous administration is preferred. Examples of such a narcotic analgesic include remifentanil and fentanyl, and particularly preferred is remifentanil (such as remifentanil hydrochloride).

In the invention, the local analgesic is not particularly limited as long as it is a local analgesic which may be used for the analgesic purpose at the general anesthesia, and examples thereof include cocaine, procaine, tetracaine, benzocaine, lidocaine, mepivacaine, bupivacaine, levobupivacaine, ropivacaine, prilocaine (propitocaine), dibucaine, and the like.

In the invention, as the muscle relaxant, a natural muscle relaxant, a synthetic muscle relaxant, or a semisynthetic muscle relaxant may be used as long as it is a muscle relaxant to be used in anesthesiological practice. However, a muscle relaxant which exhibits a muscle relaxing effect by intravenous administration is preferred. Examples of such a muscle relaxant include rocuronium, vecuronium, and suxamethonium, and particularly preferred is rocuronium (such as rocuronium bromide).

In the case of using the muscle relaxant, it is preferred to monitor the degree of muscle relaxation in a patient. The degree of muscle relaxation can be easily determined by, for example, measuring a TOF ratio using a frequently used device such as a TOF watch.

Here, the TOF ratio refers to a value obtained by a train of four method which is a method for evaluating the degree of muscle relaxation. Four separate stimuli are given at a frequency of 2 Hz. When the muscle is not relaxed, the ratio of the height of the fourth stimulus (T4) to that of the first stimulus (T1) (TOF ratio: T4/T1) is substantially 100%, and as the degree of muscle relaxation is increased, the TOF ratio is decreased. A device for measuring the TOF ratio is a TOF watch, and in Japan, a TOF watch is available from MSD, Inc. (JMDN code: 35723003; approval No: 21100BZY00162000).

According to a preferred embodiment of the dosing regimen of the invention, the dosing rate in the first step for the induction of general anesthesia is at most about 12 times faster than the dosing rate in the second step for the maintenance of general anesthesia. An appendix attached to a propofol injection (trade name: 1% Diprivan injection) currently available in Japan describes that the induction of general anesthesia is carried out at a dosing rate of 0.5 mg/kg/10 sec, and the maintenance of general anesthesia is carried out at a dosing rate of 4 to 10 mg/kg/h. When calculation was made using these dosing rates, the dosing rate in the first step for the induction of general anesthesia is 18 to 45 times faster than the dosing rate in the second step for the maintenance of general anesthesia. The fact that the time required for the induction of general anesthesia is substantially the same (about 1 to 2 minutes) and the rate for the induction of general anesthesia is slower indicates that by using the dosing regimen of the invention, a risk of overdosing at the time of induction of general anesthesia is further decreased. For example, Miller's Anesthesia, seventh edition, which is recognized as a standard of anesthetics, volume 1, page 724 describes that the most conspicuous effect during the use of propofol for the induction and maintenance of general anesthesia is a lowering of the arterial pressure at the induction of general anesthesia. That is, it may be said that the dosing regimen of the invention which reduces a risk of the occurrence of such an undesirable effect at the induction of general anesthesia is truly preferred.

Further, the dosing regimen of the invention has overcome even a problem of vasostimulant which methyl 3-[(4S)-8-bromo-1-methyl-6-(2-pyridinyl)-4H-imidazo[1,2-a][1,4]benzodiazepin-4-yl]propanoate is considered to possess potentially. For example, when Compound B is continuously intravenously administered to a non-human animal (for example, a monkey) at a dosing rate of 1 mg/kg/h to 2 mg/kg/h for several hours (for example, 4 hours to 6 hours), there may be the case where vasostimulant is observed in the neighborhood of an effective dose. However, according to the dosing regimen of the invention, even in the case where the compound is administered in an amount per kg of the body weight equal to or more than that in the preceding test examples in which vasostimulant was observed, Compound B does not cause the vasostimulant. It can be said that this is an astonishingly excellent effect provided by the dosing regimen of the invention.

### [Toxicity]

As long as the sedative comprising Compound A or a salt thereof as an active ingredient is used for the induction and maintenance of general anesthesia using the dosing regimen disclosed in the invention, side effects thereof are extremely low. For example, in the below-described Examples, the results of clinical trials in 85 cases of patients in total are shown, and hypotension whose causal relationship with methyl 3-[(4S)-8-bromo-1-methyl-6-(2-pyridinyl)-4H-imidazo[1,2-a] [1,4]benzodiazepin-4-yl]propanoate or a salt thereof, which is the active ingredient, cannot be denied, in other words, which may be caused by the administration of the compound was observed in only 9 cases (10.6%). Further, angialgia was not observed in any of the patients.

In this specification, the expression "adverse event and/or side effect" is used. The adverse event refers to every unpreferred or unintended sign (including abnormal laboratory examination values), symptom, or pathology occurring while Compound A or a salt thereof is being administered regardless of the presence or absence of the causal relationship with Compound A or a salt thereof.

Further, the side effect refers to, among the above-described adverse events, an effect which is determined such that its causal relationship with Compound A or a salt thereof cannot be denied in consideration of the conditions and past history of a test subject, a concomitant agent, a time relationship with the onset of the event, etc.

### [Application to Pharmaceutical Product]

In the case where the sedative comprising Compound A or a salt thereof as an active ingredient is used for the induction and maintenance of general anesthesia using the dosing regimen disclosed in the invention, any patient can receive the administration of the sedative as long as the patient needs general anesthesia, preferably the patient is going to undergo surgery using general anesthesia regardless of the type of surgery or the duration of surgery. For example, the 85 cases of patients in total who participated in the clinical trials, the results of which are shown in the below-described Examples, underwent surgery using general anesthesia for a different site such as the anterior neck, left forehead, lower jaw, chest, abdomen, uterus, extremities, trunk and right upper extremity, crotch, urinary tract, bladder, right hip joint, right lower extremity, etc., and it is demonstrated that the induction and maintenance of general anesthesia using the dosing regimen disclosed in the invention can be applied to any patient.

By using the sedative comprising Compound A or a salt thereof as an active ingredient for the induction and maintenance of general anesthesia using the dosing regimen disclosed in the invention, anesthesia can be induced rapidly in a patient who needs general anesthesia according to the procedure of the first step. Preferably, the loss of consciousness can be caused in a patient within about 180 seconds, more preferably within about 120 seconds, further more preferably within about 90 seconds, still further more preferably within about 70 seconds, and particularly preferably within about 60 seconds after the intravenous administration of Compound A or a salt thereof is started.

By using the sedative comprising Compound A or a salt thereof as an active ingredient for the induction and maintenance of general anesthesia using the dosing regimen disclosed in the invention, stable maintenance of anesthesia can be ensured in a patient under general anesthesia according to the procedure of the second step of the dosing regimen. Specifically, it is not necessary to perform a salvage treatment for the excessive anesthesia in the patient under general anesthesia, and further, intraoperative awakening due to light anesthesia is not caused in the patient under general anesthesia.

By using the sedative comprising Compound A or a salt thereof as an active ingredient for the induction and maintenance of general anesthesia using the dosing regimen disclosed in the invention, after stopping the administration thereof, rapid recovery from anesthesia can be caused in a patient under general anesthesia. In the below-described Examples 2 and 4, an average time until the patient opened eyes, an average time until the trachea was extubated, an average time until the patient stated the date of birth, and a time until it was determined that the patient can leave the surgery room after stopping the administration are shown, and whether the patient is an elderly adult or not, the average time until the patient opened eyes was about 15 minutes or less and the average time until it was determined that the patient can leave the surgery room was about 30 minutes or less, and therefore, it is apparent that rapid recovery from anesthesia can be obtained.

The usefulness of dosing regimen of the invention can also be confirmed by, for example, the following test (Test Example X) in addition to the four tests described in the Examples. By using the three indexes of "presence or absence of intraoperative awakening/memory", "presence or absence of salvage treatment against the sedative effect", and "presence or absence of body motion" as obtained as a result of such tests, the "function as general anesthetic" can be evaluated compositely. Further, in addition to the above, as needed, the evaluation of usefulness regarding, for example, a time from the start of the administration of Compound B until loss of consciousness, a BIS value at each point of time, a time until the patient opens eyes after the administration of Compound B is stopped, a time until extubation after the administration of Compound B is stopped, a time until the patient states the date of birth after the administration of Compound B is stopped, a time until it is determined that the patient can leave the surgery room after the administration of Compound B is stopped, or controllability of the depth of anesthesia, and evaluation of items of safety regarding, for example, adverse events, side effects, general laboratory examination (for example, hematological examination, hematobiochemical examination, or urinalysis), physical examination (for example, blood pressure/heart rate (recumbent position), respiratory rate, body temperature (for example, deep part temperature or arterial oxygen saturation), electrocardiography (for example, resting 12-lead electrocardiogram or monitor electrocardiogram), a proportion of the point of time when the systolic blood pressure is 80 mmHg or more and less than 150 mmHg to the total points of time, the number of times of using a vasopressor, observation of the administration site with an investigational new drug, or observation of the excited state, may be carried out.

### Test Example X (Induction and maintenance of general anesthesia in patients at the age of 20 or older using Compound B)

A multi-center, randomized, parallel-group, controlled study is carried out for 325 surgery patients at the age of 20 or older to undergo general anesthesia using propofol as a control in combination with a narcotic analgesic.

### [Method]

The study is carried out by allocating 325 test subjects to three groups (Group X1, Group X2, and Group X3) according to the description in the following Table 1.

### [Table 1]

**Table 1**

| Group | X1 | X2 | X3 |
|---|---|---|---|
| Dosing rate of Compound B (mg/kg/h) | 6 | 12 | |
| Dose of propofol (mg/kg) | | | 2.0-2.5 (slowly) |
| Test subjects | 130 | 130 | 65 |

After the test subjects are subjected to an analgesic treatment by intravenous continuous administration of remifentanil hydrochloride (see the following a-1), the intravenous administration of Compound B or propofol (see the following a-2) is started. After loss of consciousness in the test subjects is confirmed, rocuronium bromide is intravenously administered (see the following a-3), and the dosing rate of Compound B or propofol is also changed (see the following a-4). After confirming that a sufficient muscle relaxing effect is obtained, endotracheal intubation is performed. During the surgery, the intravenous continuous administration of remifentanil hydrochloride (see the following a-5) and the intravenous continuous administration of Compound B or propofol are continued, and rocuronium bromide is administered (see the following a-6), as needed. After completion of the surgery, the intravenous continuous administration of remifentanil hydrochloride and Compound B or propofol is stopped.

### (a-1)

The dosing rate of remifentanil hydrochloride is set to 0.25 to 0.5 µg/kg/min. Incidentally, it is to be noted that the dose of remifentanil hydrochloride can be appropriately increased or decreased according to the age or general conditions of the test subject.

### (a-2)

The dosing rate of Compound B is set according to the description in the above Table 1. Incidentally, it is to be noted that in the Group X3, propofol which is the control is used in place of Compound B and is slowly administered at a dose of 2.0 to 2.5 mg/kg.

### (a-3)

The dose of rocuronium bromide is set to 0.6 to 0.9 mg/kg. Incidentally, it is to be noted that the dose of rocuronium bromide can be appropriately increased or decreased according to the age or general conditions of the test subject.

### (a-4)

The dosing rate of Compound B is set to 1 mg/kg/h. Incidentally, it is to be noted that the dosing rate of Compound B can be appropriately increased or decreased within a range of 2 mg/kg/h at maximum while observing the general conditions of the test subject until completion of the surgery. Further, the dosing rate of propofol is set to 4 to 10 mg/kg/h, and it is to be noted that the dosing rate of propofol can also be appropriately increased or decreased while observing the general conditions of the test subject until completion of the surgery.

### (a-5)

The dosing rate of remifentanil hydrochloride is set to 0.25 µg/kg/min. Incidentally, it is to be noted that the dosing rate of remifentanil hydrochloride can be appropriately increased or decreased within a range of 2.0 µg/kg/min at maximum while observing the general conditions of the test subject. It is to be noted that during light anesthesia, the single intravenous administration of remifentanil hydrochloride can be additionally performed at a dose of 0.5 to 1.0 µg/kg.

### (a-6)

It is to be noted that during the surgery, rocuronium bromide can be intravenously administered at a dose of 0.1 to 0.2 mg/kg, or can be intravenously and continuously administered at a dosing rate of 7 µg/kg/min which is used as a standard, as needed.

The dosing regimen of the invention, the usefulness of which has been confirmed by carrying out the studies described in the Examples or the above-described study, may be described by providing appropriate items regarding efficacy/effect, dosage and administration, and the like in at least one of an instruction, a manual, an appendix, and a product label (including one corresponding to a label or labeling or labelling in U.S.A.) to be attached to, for example, a pharmaceutical composition comprising as an active ingredient, the compound or a salt thereof (preferably Compound B). Though such examples are not limited to this, examples thereof include the description of the following Case 1 or 2.

### (Case 1)

### [Efficacy/Effect]

### Induction and Maintenance of general anesthesia

### [Dosage/Administration]

### (1) Induction

In general, remimazolam is intravenously administered at a rate of 6 mg/kg/h to an adult patient while observing the general conditions of the patient until sleeping is obtained.

### (2) Maintenance

The dosing rate is adjusted so as to obtain an adequate depth of anesthesia while observing the general conditions of a patient. In general, for the adult patient, the adequate depth of anesthesia is obtained at a dosing rate of remimazolam of 0.4 to 1 mg/kg/h.

Further, an analgesic (for example, a narcotic analgesic or a local analgesic) is to be used in combination.

### (Case 2)

### [Efficacy/Effect]

### Induction and Maintenance of general anesthesia

### [Dosage/Administration]

### (1) Induction

In general, remimazolam is intravenously administered at a rate of 12 mg/kg/h to an adult patient while observing the general conditions of the patient until sleeping is obtained.

### (2) Maintenance

The dosing rate is adjusted so as to obtain an adequate depth of anesthesia while observing the general conditions of a patient. In general, for the adult patient, the adequate depth of anesthesia is obtained at a dosing rate of remimazolam of 0.4 to 1 mg/kg/h.

Further, an analgesic (for example, a narcotic analgesic or a local analgesic) is to be used in combination.

In the invention, the "container" means one enclosing immediately Compound A or a salt thereof as the active ingredient, and it may also be called an "intermediate container", an "immediate wrapper", or an "inner seals", etc. In general, examples of the container which is used include a can, a bottle, a box, an ampoule, a vial, a tube, a unit dose container for eyedrop medicines, paper, a cloth, a plastic film, a plastic bag, an SP sheet, a PTP sheet, a plastic container, and the like. However, in the invention, a container for injection, such as an ampoule or a vial, is preferred.

All of the technical or scientific terminologies and abbreviates used in this specification have the same meanings as those ordinarily understood by one skilled in the art who belongs to the field of the invention, unless otherwise indicated.

This application claims priority to Japanese Patent Application No. 2012-192081 filed on August 31, 2012, the entire contents of which are incorporated by reference herein.

The entire contents of all of the patent documents and the non-patent documents or the references explicitly cited in this specification are incorporated herein by reference as part of this specification.

### [Examples]

Hereinafter, the invention will be described in detail with reference to Examples and Biological Examples, however, the invention is not limited thereto.

In the following studies, as Compound B to be administered, a pharmaceutical preparation described in the below-mentioned Preparation Example was used.

Further, in the following studies, in order to confirm safety, adverse events and side effects were observed, and also general laboratory examination (hematological examination, hematobiochemical examination and urinalysis), physical examination (blood pressure, pulse rate, respiratory rate, body temperature, blood oxygen saturation level and end-tidal carbon dioxide level), electrocardiography, etc. were carried out according to the procedures commonly used by those skilled in the art.

The term "BMI" described in tables denotes "body mass index" and is a physique index which represents the degree of obesity in humans and is calculated from the relationship between body weight and body height. BMI can be calculated according to the formula: {BMI = body weight (kg) / (body height (m))²}.

### Example 1 (Induction of general anesthesia in non-elderly adults using Compound B)

A non-blind study was carried out for 25 surgery patients at the age of 20 or older and younger than 65 to undergo general anesthesia using Compound B in combination with a narcotic analgesic.

### [Method]

The study was carried out by allocating 25 test subjects to four groups (Group A1, Group A2, Group A3 and Group A4) according to the description in the following Table 2.

### [Table 2]

**Table 2**

| Group | | A1 | A2 | A3 | A4 |
|---|---|---|---|---|---|
| Dosing rate of Compound B (mg/kg/h) | | 6 | 12 | 21 | 30 |
| Number of test subjects (patients) | | 5 | 10 | 5 | 5 |
| Gender of test subjects (patients) | Male | 2 | 1 | 4 | 3 |
| | Female | 3 | 9 | 1 | 2 |

At 5 minutes before the start of the administration of Compound B, the intravenous continuous administration of remifentanil hydrochloride (see the following 1-1) was started, and after 5 minutes passed, the intravenous continuous administration of Compound B (see the following 1-2) was started. After loss of consciousness in the test subjects was confirmed, rocuronium bromide was intravenously administered (see the following 1-3) and also the dosing rate of Compound B was changed (see the following 1-4). After confirming that the TOF ratio reached 0 by using a TOF Watch, endotracheal intubation was performed, and the intravenous continuous administration of remifentanil hydrochloride and Compound B was stopped. After endotracheal intubation, sevoflurane was used for the maintenance of general anesthesia during the surgery.
(1-1) The dosing rate of remifentanil hydrochloride was set to 0.25 µg/kg/min.
(1-2) The dosing rate of Compound B was set according to the description in the above Table 2.
(1-3) The dose of rocuronium bromide was set to 0.6 mg/kg. The dose of rocuronium bromide can be increased or decreased appropriately according to the age or symptoms of the test subject, however, the upper limit thereof was set to 0.9 mg/kg.
(1-4) The dosing rate of Compound B was set to 1 mg/kg/h. The dosing rate of Compound B can be increased or decreased appropriately according to the conditions of the test subject, and it was determined that when a BIS value exceeds 53 or signs of awakening (body motion, change in blood pressure or pulse rate, lacrimation, sweating, etc.) are observed, the dosing rate of Compound B can be increased to a maximum of 2.5 mg/kg/h, and when it is confirmed that a BIS value is 53 or less and also signs of awakening are not observed, the dosing rate of Compound B can be carefully decreased.

### [Results]

The results obtained in Example 1 are shown in the following Table 3 (the ± numerical value in each parenthesis additionally shown for the average age, average body height, average body weight and BMI represents a standard error, and as for the time until loss of consciousness, an average value and a range of actual values (in parenthesis) of the times in the patients (25 patients) in compliance with the study protocol are shown).

In the step for the induction of general anesthesia, by intravenously and continuously administering Compound B to the patients at a dosing rate of 6, 12, 21 or 30 mg/kg/h, the patients lost consciousness in 108.0 sec, 70.3 sec, 65.8 sec or 65.4 sec (each of which is an average value), respectively. The completion rate of endotracheal intubation was 100%. Only one patient in Group A2 developed hypotension as a side effect, which shows that the incidence of hypotension was not increased as the dosing rate of Compound B was increased. Further, other side effects to be concerned or abnormal laboratory data were not observed.

### [Table 3]

**Table 3**

| Group | | A1 | A2 | A3 | A4 |
|---|---|---|---|---|---|
| Average age (years old) | | 53.2 (±9.5) | 49.3 (±14.5) | 51.4 (±14.0) | 50.4 (±10.1) |
| Average body height (cm) | | 161.7 (±1.69) | 156.9 (±6.73) | 163.4 (±5.22) | 161.7 (±13.11) |
| Average body weight (kg) | | 59.4 (±3.40) | 56.3 (±10.30) | 71.5 (±22.67) | 61.5 (±13.20) |
| BMI | | 22.75 (±1.52) | 22.88 (±4.04) | 27.02 (±9.65) | 23.28 (±2.77) |
| Time until loss of consciousness (sec) | | 108.0 (97-117) | 70.3 (53-84) | 65.8 (61-73) | 65.4 (54-83) |
| Number of test subjects who developed hypotension (patients) | Adverse event | 0 | 3 | 3 | 1 |
| | Side effect | 0 | 1 | 0 | 0 |

### Example 2 (Induction and maintenance of general anesthesia in non-elderly adults using Compound B)

A non-blind study was carried out for 30 surgery patients at the age of 20 or older and younger than 65 to undergo general anesthesia using Compound B in combination with a narcotic analgesic.

### [Method]

The study was carried out by allocating all of the 30 test subjects to one group (Group B).

At 5 minutes before the start of the administration of Compound B, the intravenous continuous administration of remifentanil hydrochloride (see the following 2-1) was started, and after 5 minutes passed, the intravenous continuous administration of Compound B (see the following 2-2) was started. After loss of consciousness in the test subjects was confirmed, rocuronium bromide was intravenously administered (see the following 2-3) and also the dosing rate of Compound B was changed (see the following 2-4). After confirming that the TOF ratio reached 0 by using a TOF Watch, endotracheal intubation was performed. During the surgery, the intravenous continuous administration of remifentanil hydrochloride (see the following 2-5) and the intravenous continuous administration of Compound B (see the following 2-6) were continued, and rocuronium bromide was administered (see the following 2-7) as needed. After completion of the surgery, it was confirmed that the TOF ratio increased to 0.9 or higher by using a TOF Watch, and then, the intravenous continuous administration of remifentanil hydrochloride and Compound B was stopped.
(2-1) The dosing rate of remifentanil hydrochloride was set to 0.25 µg/kg/min.
(2-2) The dosing rate of Compound B was set to 12 mg/kg/h.
(2-3) The dose of rocuronium bromide was set to 0.6 mg/kg. The dose of rocuronium bromide can be increased or decreased appropriately according to the age or symptoms of the test subject, however, the upper limit thereof was set to 0.9 mg/kg.
(2-4) The dosing rate of Compound B was set to 1 mg/kg/h. The dosing rate of Compound B can be increased or decreased appropriately according to the conditions of the test subject, and it was determined that when a BIS value exceeds 53 or signs of awakening (body motion, change in blood pressure or pulse rate, lacrimation, sweating, etc.) are observed, the dosing rate of Compound B can be increased to a maximum of 2.5 mg/kg/h, and when it is confirmed that a BIS value is 53 or less and also signs of awakening are not observed, the dosing rate of Compound B can be carefully decreased.
(2-5) The dosing rate of remifentanil hydrochloride was set to 0.25 µg/kg/min. It was determined that the dosing rate of remifentanil hydrochloride can be increased by 25% to 100% (with the proviso that the dosing rate does not exceed a maximum of 2.0 µg/kg/min) at intervals of from 2 to 5 minutes, or can be decreased by 25% to 50% while observing the general conditions of the test subject. It was also determined that during light anesthesia, the single intravenous administration of remifentanil hydrochloride can be additionally performed at a dose of from 0.5 to 1.0 µg/kg at intervals of from 2 to 5 minutes.
(2-6) The dosing rate of Compound B was set to 1 mg/kg/h. The dosing rate of Compound B can be increased or decreased appropriately according to the conditions of the test subject, and it was determined that when a BIS value exceeds 53 or signs of awakening (body motion, change in blood pressure or pulse rate, lacrimation, sweating, etc.) are observed, the dosing rate of Compound B can be increased to a maximum of 2.5 mg/kg/h, and when it is confirmed that a BIS value is 53 or less and also signs of awakening are not observed, the dosing rate of Compound B can be carefully decreased.
(2-7) It was determined that rocuronium bromide can be intravenously administered at a dose of from 0.1 to 0.2 mg/kg or intravenously and continuously administered at a dosing rate of 7 µg/kg/min which is used as a standard.

### [Results]

The results obtained in Example 2 are shown in the following Table 4 (the ± numerical value in each parenthesis additionally shown for the average age, average body height, average body weight and BMI represents a standard error, and as for the time until loss of consciousness, an average value and a range of actual values (in parenthesis) of the times in the patients (25 patients) in compliance with the study protocol are shown).

In the step for the induction of general anesthesia, by intravenously and continuously administering Compound B to the patients at a dosing rate of 12 mg/kg/h, the patients lost consciousness in 72.8 sec (which is an average value). The completion rate of endotracheal intubation was 100%.

In the step for the maintenance of general anesthesia, the dosing rate of Compound B was set to 1 mg/kg/h at the beginning, and thereafter, the dosing rate of Compound B was appropriately adjusted while observing the conditions of the test subject such as a BIS value, whereby the surgery could be completed without performing a salvage treatment for sedation in all of the test subjects. Further, signs of awakening during the surgery were not observed and it was confirmed that the test subjects had no recollection during anesthesia.

The number of patients who developed hypotension as a side effect was three, which accounts for 10% of the test subjects in Group B. Further, other side effects to be concerned or abnormal laboratory data were not observed.

The average of the dosing periods of Compound B of 30 cases in the step for the maintenance of general anesthesia was 167.0 ± 81.3 min (the shortest dosing period: 4.7 min and the longest dosing period: 329.7 min). The maintenance of general anesthesia using Compound B was safely and effectively performed throughout these dosing periods.

### [Table 4]

**Table 4**

| Group | | B |
|---|---|---|
| Average age (years old) | | 45.8 (±11.4) |
| Average body height (cm) | | 157.3 (±7.69) |
| Average body weight (kg) | | 55.9 (±10.58) |
| BMI | | 22.64 (±4.37) |
| Time until loss of consciousness (sec) | | 72.8 (48-120) |
| Number of test subjects who developed hypotension (patients) | Adverse event | 3 |
| | Side effect | 3 |

In order to confirm the rapidity of recovery from anesthesia after stopping the administration, for the patients (24 patients) in compliance with the study protocol, a time until the patient opened eyes, a time until the trachea was extubated, a time until the patient stated the date of birth, and a time until it was determined that the patient can leave the surgery room, after stopping the administration of Compound B were measured, and the following results were obtained.
- Average time until the patient opened eyes (min): 13.9
- Average time until the trachea was extubated (min): 16.4
- Average time until the patient stated the date of birth (min): 21.5
- Average time until the patient were allowed to leave the surgery room (min): 25.5

These results showed that Compound B allows rapid recovery from anesthesia after stopping the administration of Compound B.

### Example 3 (Induction of general anesthesia in elderly adults using Compound B)

A non-blind study was carried out for 20 surgery patients at the age of 65 or older to undergo general anesthesia using Compound B in combination with a narcotic analgesic.

### [Method]

The study was carried out by allocating 20 test subjects to three groups (Group C1, Group C2 and Group C3) according to the description in the following Table 5.

### [Table 5]

**Table 5**

| Group | | C1 | C2 | C3 |
|---|---|---|---|---|
| Dosing rate of Compound B (mg/kg/h) | | 4 | 8 | 12 |
| Number of test subjects (patients) | | 10 | 5 | 5 |
| Gender of test subjects (patients) | Male | 6 | 3 | 3 |
| | Female | 4 | 2 | 2 |

At 5 minutes before the start of the administration of Compound B, the intravenous continuous administration of remifentanil hydrochloride (see the following 3-1) was started, and after 5 minutes passed, the intravenous continuous administration of Compound B (see the following 3-2) was started. After loss of consciousness in the test subjects was confirmed, rocuronium bromide was intravenously administered (see the following 3-3) and also the dosing rate of Compound B was changed (see the following 3-4). After confirming that the TOF ratio reached 0 by using a TOF Watch, endotracheal intubation was performed, and the intravenous continuous administration of remifentanil hydrochloride and Compound B was stopped. After endotracheal intubation, sevoflurane was used for the maintenance of general anesthesia during the surgery.
(3-1) The dosing rate of remifentanil hydrochloride was set to 0.25 µg/kg/min.
(3-2) The dosing rate of Compound B was set according to the description in the above Table 5.
(3-3) The dose of rocuronium bromide was set to 0.6 mg/kg. The dose of rocuronium bromide can be increased or decreased appropriately according to the age or symptoms of the test subject, however, the upper limit thereof was set to 0.9 mg/kg.
(3-4) The dosing rate of Compound B was set to 1 mg/kg/h. The dosing rate of Compound B can be increased or decreased appropriately according to the conditions of the test subject, and it was determined that when a BIS value exceeds 53 or signs of awakening (body motion, change in blood pressure or pulse rate, lacrimation, sweating, etc.) are observed, the dosing rate of Compound B can be increased to a maximum of 2.5 mg/kg/h, and when it is confirmed that a BIS value is 53 or less and also signs of awakening are not observed, the dosing rate of Compound B can be carefully decreased.

### [Results]

The results obtained in Example 3 are shown in the following Table 6 (the ± numerical value in each parenthesis additionally shown for the average age, average body height, average body weight and BMI represents a standard error, and as for the time until loss of consciousness, an average value and a range of actual values (in parenthesis) of the times in the patients (Group C1: 9 patients, Group C2: 4 patients and Group C3: 5 patients) in compliance with the study protocol are shown).

In the step for the induction of general anesthesia, by intravenously and continuously administering Compound B to the patients at a dosing rate of 4, 8 or 12 mg/kg/h, the patients lost consciousness in 115.2 sec, 72.5 sec or 57.6 sec (each of which is an average value), respectively. The completion rate of endotracheal intubation was 100%. The number of patients who developed hypotension as a side effect was one in each Group, which shows that the incidence of hypotension was not increased as the dosing rate of Compound B was increased. Further, other side effects to be concerned or abnormal laboratory data were not observed.

### [Table 6]

**Table 6**

| Group | | C1 | C2 | C3 |
|---|---|---|---|---|
| Average age (years old) | | 72.9 (±5.4) | 75.0 (±4.2) | 74.2 (±5.9) |
| Average body height (cm) | | 157.9 (±8.00) | 153.5 (±6.33) | 152.8 (±10.61) |
| Average body weight (kg) | | 57.2 (±11.77) | 58.3 (±6.93) | 57.9 (±5.43) |
| BMI | | 22.78 (±3.35) | 24.66 (±1.14) | 25.05 (±4.02) |
| Time until loss of consciousness (sec) | | 115.2 (67-165) | 72.5 (58-87) | 57.6 (43-68) |
| Number of test subjects who developed hypotension (patients) | Adverse event | 2 | 3 | 2 |
| | Side effect | 1 | 1 | 1 |

### Example 4 (Induction and maintenance of general anesthesia in elderly adults using Compound B)

A non-blind study was carried out for 10 surgery patients at the age of 65 or older to undergo general anesthesia using Compound B in combination with a narcotic analgesic.

### [Method]

The study was carried out by allocating all of the 10 test subjects to one group (Group D).

At 5 minutes before the start of the administration of Compound B, the intravenous continuous administration of remifentanil hydrochloride (see the following 4-1) was started, and after 5 minutes passed, the intravenous continuous administration of Compound B (see the following 4-2) was started. After loss of consciousness in the test subjects was confirmed, rocuronium bromide was intravenously administered (see the following 4-3) and also the dosing rate of Compound B was changed (see the following 4-4). After confirming that the TOF ratio reached 0 by using a TOF Watch, endotracheal intubation was performed. During the surgery, the intravenous continuous administration of remifentanil hydrochloride (see the following 4-5) and the intravenous continuous administration of Compound B (see the following 4-6) were continued, and rocuronium bromide was administered (see the following 4-7) as needed. After completion of the surgery, it was confirmed that the TOF ratio increased to 0.9 or higher by using a TOF Watch, and then, the intravenous continuous administration of remifentanil hydrochloride and Compound B was stopped.
(4-1) The dosing rate of remifentanil hydrochloride was set to 0.25 µg/kg/min.
(4-2) The dosing rate of Compound B was set to 4 mg/kg/h.
(4-3) The dose of rocuronium bromide was set to 0.6 mg/kg. The dose of rocuronium bromide can be increased or decreased appropriately according to the age or symptoms of the test subject, however, the upper limit thereof was set to 0.9 mg/kg.
(4-4) The dosing rate of Compound B was set to 1 mg/kg/h. The dosing rate of Compound B can be increased or decreased appropriately according to the conditions of the test subject, and it was determined that when a BIS value exceeds 53 or signs of awakening (body motion, change in blood pressure or pulse rate, lacrimation, sweating, etc.) are observed, the dosing rate of Compound B can be increased to a maximum of 2.5 mg/kg/h, and when it is confirmed that a BIS value is 53 or less and also signs of awakening are not observed, the dosing rate of Compound B can be carefully decreased.
(4-5) The dosing rate of remifentanil hydrochloride was set to 0.25 µg/kg/min. It was determined that the dosing rate of remifentanil hydrochloride can be increased by 25% to 100% (with the proviso that the dosing rate does not exceed a maximum of 2.0 µg/kg/min) at intervals of from 2 to 5 minutes, or can be decreased by 25% to 50% while observing the general conditions of the test subject. It was also determined that during light anesthesia, the single intravenous administration of remifentanil hydrochloride can be additionally performed at a dose of from 0.5 to 1.0 µg/kg at intervals of from 2 to 5 minutes.
(4-6) The dosing rate of Compound B was set to 1 mg/kg/h. The dosing rate of Compound B can be increased or decreased appropriately according to the conditions of the test subject, and it was determined that when a BIS value exceeds 53 or signs of awakening (body motion, change in blood pressure or pulse rate, lacrimation, sweating, etc.) are observed, the dosing rate of Compound B can be increased to a maximum of 2.5 mg/kg/h, and when it is confirmed that a BIS value is 53 or less and also signs of awakening are not observed, the dosing rate of Compound B can be carefully decreased.
(4-7) It was determined that rocuronium bromide can be intravenously administered at a dose of from 0.1 to 0.2 mg/kg or intravenously and continuously administered at a dosing rate of 7 µg/kg/min which is used as a standard.

### [Results]

The results obtained in Example 4 are shown in the following Table 7 (the ± numerical value in each parenthesis additionally shown for the average age, average body height, average body weight and BMI represents a standard error, and as for the time until loss of consciousness, an average value and a range of actual values (in parenthesis) of the times in the patients (7 patients) in compliance with the study protocol are shown).

In the step for the induction of general anesthesia, by intravenously and continuously administering Compound B to the patients at a dosing rate of 4 mg/kg/h, the patients lost consciousness in 100.3 sec (which is an average value). The completion rate of endotracheal intubation was 100%.

In the step for the maintenance of general anesthesia, the dosing rate of Compound B was set to 1 mg/kg/h at the beginning, and thereafter, the dosing rate of Compound B was appropriately adjusted while observing the conditions of the test subject such as a BIS value, whereby the surgery could be completed without performing a salvage treatment for sedation in all of the test subjects. Further, signs of awakening during the surgery were not observed and it was confirmed that the test subjects had no recollection during anesthesia.

The number of patients who developed hypotension as a side effect was two, which accounts for 20% of the test subjects in Group D. Further, other side effects to be concerned or abnormal laboratory data were not observed.

The average of the dosing periods of Compound B of 10 cases in the step for the maintenance of general anesthesia was 229.6 ± 151.3 min (the shortest dosing period: 57.0 min and the longest dosing period: 621.7 min). The maintenance of general anesthesia using Compound B was safely and effectively performed throughout these dosing periods.

### [Table 7]

**Table 7**

| Group | | D |
|---|---|---|
| Average age (years old) | | 75.1 (±7.4) |
| Average body height (cm) | | 153.4 (±10.98) |
| Average body weight (kg) | | 56.8 (±8.52) |
| BMI | | 24.10 (±2.48) |
| Time until loss of consciousness (sec) | | 100.3 (70-135) |
| Number of test subjects who developed hypotension (patients) | Adverse event | 3 |
| | Side effect | 2 |

In order to confirm the rapidity of recovery from anesthesia after stopping the administration, for the patients (7 patients) in compliance with the study protocol, a time until the patient opened eyes, a time until the trachea was extubated, a time until the patient stated the date of birth, and a time until it was determined that the patient can leave the surgery room, after stopping the administration of Compound B were measured, and the following results were obtained.
- Average time until the patient opened eyes (min): 10.9
- Average time until the trachea was extubated (min): 13.6
- Average time until the patient stated the date of birth (min): 18.5
- Average time until the patient were allowed to leave the surgery room (min): 19.1

These results showed that Compound B allows rapid recovery from anesthesia after stopping the administration of Compound B.

### Formulation Example 1 (Injection) (ampoule)

Methyl 3-[(4S)-8-bromo-1-methyl-6-(2-pyridinyl)-4H-imidazo[1,2-a][1,4]benzodiazepin-4-yl]propanoate benzenesulfonate (89.76 g), maltose (473 g), and polysorbate 80 (5.5 g) were mixed in a conventional manner, and a pH adjusting agent and water for injection were added thereto, whereby a solution (5.5 L) at a pH of 3.0 was obtained. The obtained solution was sterilized in a conventional manner and filled in ampoules at 5 mL per ampoule, followed by lyophilization in a conventional manner, whereby 1,000 ampoules each comprising 81.6 mg of methyl 3-[(4S)-8-bromo-1-methyl-6-(2-pyridinyl)-4H-imidazo[1,2-a][1,4]benzodiazepin-4-yl]propanoate benzenesulfonate (60 mg in terms of the amount of methyl 3-[(4S)-8-bromo-1-methyl-6-(2-pyridinyl)-4H-imidazo[1,2-a] [1,4]benzodiazepin-4-yl]propanoate) were obtained.

### Formulation Example 2 (Injection) (vial)

Methyl 3-[(4S)-8-bromo-1-methyl-6-(2-pyridinyl)-4H-imidazo[1,2-a][1,4]benzodiazepin-4-yl]propanoate benzenesulfonate (89.76 g), maltose (473 g), and polysorbate 80 (5.5 g) were mixed in a conventional manner, and a pH adjusting agent and water for injection were added thereto, whereby a solution (5.5 L) at a pH of 3.0 was obtained. The obtained solution was sterilized in a conventional manner and filled in vials at 5 mL per vial, followed by lyophilization in a conventional manner, whereby 1,000 vials each comprising 81.6 mg of methyl 3-[(4S)-8-bromo-1-methyl-6-(2-pyridinyl)-4H-imidazo[1,2-a] [1,4]benzodiazepin-4-yl]propanoate benzenesulfonate (60 mg in terms of the amount of methyl 3-[(4S)-8-bromo-1-methyl-6-(2-pyridinyl)-4H-imidazo[1,2-a] [1,4]benzodiazepin-4-yl]propanoate) were obtained.

### [Industrial Applicability]

The sedative employing the dosing regimen disclosed in the invention is safe, can rapidly induce general anesthesia in a patient and stably maintain the state of general anesthesia, and allows rapid recovery from anesthesia in a patient after stopping the administration thereof, and therefore is useful for the induction and/or maintenance of general anesthesia.

## Claims

1. A compound for use in sedation, wherein the compound is methyl 3-[(4S)-8-bromo-1-methyl-6-(2-pyridinyl)-4H-imidazo[1,2-a][1,4]benzodiazepin-4-yl]propanoate or a salt thereof, and the dosing rate of the compound by intravenous administration is about 0.3 mg/hour/kg to about 40 mg/hour/kg in a patient, the use comprises the induction and maintenance of general anesthesia, and the intravenous administration includes a two-step administration process, which comprises: a first step for the induction of general anesthesia, in which the dosing rate of the compound by intravenous administration is about 3 mg/hour/kg to about 40 mg/hour/kg; and a second step for the maintenance of general anesthesia, in which the dosing rate of the compound by intravenous administration is about 0.3 mg/hour/kg to about 2.5 mg/hour/kg.

2. The compound for use according to claim 1, wherein the compound is methyl 3-[(4S)-8-bromo-1-methyl-6-(2-pyridinyl)-4H-imidazo[1,2-a][1,4]benzodiazepin-4-yl]propanoate benzenesulfonate.

3. The compound for use according to claim 1 or 2, wherein the use is in combination with a narcotic analgesic and optionally a muscle relaxant.

4. The compound for use according to claim 1 or 2, wherein in the first step, the dosing rate is about 4 mg/hour/kg to about 30 mg/hour/kg, and the patient is (1) 20 years old or older and younger than 65 years old or (2) 65 years old or older.

5. A compound for use in inducing loss of consciousness in a patient within about 180 seconds after intravenous administration of the compound is started, wherein the compound is methyl 3-[(4S)-8-bromo-1-methyl-6-(2-pyridinyl)-4H-imidazo[1,2-a][1,4]benzodiazepin-4-yl]propanoatebenzenesulfonate, and wherein the use is in combination with a narcotic analgesic and optionally a muscle relaxant for the induction and maintenance of general anesthesia, the intravenous administration of the compound comprises a two-step administration process, and in a first step for the induction of general anesthesia, the dosing rate of the compound by intravenous administration is about 4 mg/hour/kg to about 30 mg/hour/kg in the patient and in a second step for the maintenance of general anesthesia, the dosing rate of the compound by intravenous administration is about 0.4 mg/hour/kg to about 2 mg/hour/kg in the patient.

6. The compound for use according to claim 3 or 5, wherein the narcotic analgesic is remifentanil, and/or the muscle relaxant is rocuronium bromide.

7. The compound for use according to claim 1, 5 or 6, wherein the incidence rate of hypotension as a side effect is less than 15%.

8. The compound for use according to claim 2, wherein the use is in combination with a narcotic analgesic and optionally a muscle relaxant, and in the first step, the dosing rate of the compound by intravenous administration is about 6 or 12 mg/hour/kg in the patient and in the second step , the dosing rate of the compound by intravenous administration is about 0.4 mg/hour/kg to about 1 mg/hour/kg in the patient.

## Patentansprüche

1. Verbindung zur Verwendung beim Sedieren, wobei die Verbindung Methyl-3-[(4S)-8-brom-1-methyl-6-(2-pyridinyl)-4H-imidazo[1,2-a][1,4]benzodiazepin-4-yl]propanoat oder ein Salz davon ist und die Dosierungsrate der Verbindung durch intravenöse Verabreichung etwa 0,3 mg/Stunde/kg bis etwa 40 mg/Stunde/kg bei einem Patienten ist, die Verwendung die Einleitung und Aufrechterhaltung einer allgemeinen Anästhesie umfasst und die intravenöse Verabreichung einen zweistufigen Verabreichungsprozess einschließt, der Folgendes umfasst: einen ersten Schritt zur Einleitung einer allgemeinen Anästhesie, wobei die Dosierungsrate der Verbindung durch intravenöse Verabreichung etwa 3 mg/Stunde/kg bis etwa 40 mg/Stunde/kg ist; und einen zweiten Schritt zur Aufrechterhaltung einer allgemeinen Anästhesie, wobei die Dosierungsrate der Verbindung durch intravenöse Verabreichung etwa 0,3 mg/Stunde/kg bis etwa 2,5 mg/Stunde/kg ist.

2. Verbindung zur Verwendung nach Anspruch 1, wobei die Verbindung Methyl-3-[(4S)-8-brom-1-methyl-6-(2-pyridinyl)-4H-imidazo[1,2-a][1,4]benzodiazepin-4-yl]propanoatbenzolsulfonat ist.

3. Verbindung zur Verwendung nach Anspruch 1 oder 2, wobei die Verwendung in Kombination mit einem narkotischen Analgetikum und optional einem Muskelrelaxans erfolgt.

4. Verbindung zur Verwendung nach Anspruch 1 oder 2, wobei die Dosierungsrate in der ersten Stufe etwa 4 mg/Stunde/kg bis etwa 30 mg/Stunde/kg ist und der Patient (1) 20 Jahre alt oder älter und jünger als 65 Jahre alt oder (2) 65 Jahre alt oder älter ist.

5. Verbindung zur Verwendung beim Einleiten von Bewusstseinsverlust bei einem Patienten innerhalb von etwa 180 Sekunden nach Beginn der intravenösen Verabreichung der Verbindung, wobei die Verbindung Methyl-3-[(4S)-8-brom-1-methyl-6-(2-pyridinyl)-4H-imidazo[1,2-a][1,4]benzodiazepin-4-yl]propanoatebenzensulfonat ist, und wobei die Verwendung in Kombination mit einem narkotischen Analgetikum und optional einem Muskelrelaxans zur Einleitung und Aufrechterhaltung einer allgemeinen Anästhesie erfolgt, die intravenöse Verabreichung der Verbindung einen zweistufigen Verabreichungsprozess umfasst, und in einem ersten Schritt zum Einleiten einer allgemeinen Anästhesie die Dosierungsrate der Verbindung durch intravenöse Verabreichung etwa 4 mg/Stunde/kg bis etwa 30 mg/Stunde/kg bei dem Patienten ist und in einem zweiten Schritt zur Aufrechterhaltung einer allgemeinen Anästhesie die Dosierungsrate der Verbindung durch intravenöse Verabreichung etwa 0,4 mg/Stunde/kg bis etwa 2 mg/Stunde/kg bei dem Patienten ist.

6. Verbindung zur Verwendung nach Anspruch 3 oder 5, wobei das narkotische Analgetikum Remifentanil ist und/oder das Muskelrelaxans Rocuroniumbromid ist.

7. Verbindung zur Verwendung nach Anspruch 1, 5 oder 6, wobei die Inzidenzrate von Hypotonie als Nebenwirkung weniger als 15 % ist.

8. Verbindung zur Verwendung nach Anspruch 2, wobei die Verwendung in Kombination mit einem narkotischen Analgetikum und optional einem Muskelrelaxans ist und in der ersten Stufe die Dosierungsrate der Verbindung durch intravenöse Verabreichung etwa 6 oder 12 mg/Stunde/kg bei dem Patienten ist und in der zweiten Stufe die Dosierungsrate der Verbindung durch intravenöse Verabreichung etwa 0,4 mg/Stunde/kg bis etwa 1 mg/Stunde/kg bei dem Patienten ist.

## Revendications

1. Composé destiné à être utilisé dans la sédation, dans lequel le composé est le méthyl 3-[(4S)-8-bromo-1-méthyl-6-(2-pyridinyl)-4H-imidazo[1,2-a][1,4]benzodiazepin-4-yl]propanoate ou un sel de celui-ci, et le taux de dosage du composé par administration intraveineuse est d'environ 0,3 mg/heure/kg à environ 40 mg/heure/kg chez un patient, l'utilisation comprend l'induction et le maintien d'une anesthésie générale, et l'administration intraveineuse comprend un procédé d'administration en deux étapes, qui comprend : une première étape pour l'induction d'une anesthésie générale, dans laquelle le taux de dosage du composé par administration intraveineuse est d'environ 3 mg/heure/kg à environ 40 mg/heure/kg ; et une seconde étape pour le maintien de l'anesthésie générale, dans laquelle le taux de dosage du composé par administration intraveineuse est d'environ 0,3 mg/heure/kg à environ 2,5 mg/heure/kg.

2. Composé destiné à être utilisé selon la revendication 1, dans lequel le composé est le méthyl 3-[(4S)-8-bromo-1-méthyl-6-(2-pyridinyl)-4H-imidazo[1,2-a] [1,4]benzodiazepin-4-yl]propanoate benzenesulfonate.

3. Composé destiné à être utilisé selon la revendication 1 ou 2, dans lequel l'utilisation est en combinaison avec un analgésique narcotique et éventuellement un myorelaxant.

4. Composé destiné à être utilisé selon la revendication 1 ou 2, dans lequel dans la première étape, le taux de dosage est d'environ 4 mg/heure/kg à environ 30 mg/heure/kg, et le patient a (1) 20 ans ou plus et moins de 65 ans ou (2) 65 ans ou plus.

5. Composé destiné à être utilisé pour induire une perte de conscience chez un patient dans les 180 secondes environ suivant le début de l'administration intraveineuse du composé, dans lequel le composé est le méthyl 3-[(4S)-8-bromo-1-méthyl-6-(2-pyridinyl)-4H-imidazo[1,2-a][1,4]benzodiazepin-4-yl]propanoatebenzenesulfonate, et dans lequel l'utilisation est en combinaison avec un analgésique narcotique et éventuellement un myorelaxant pour l'induction et le maintien d'une anesthésie générale, l'administration intraveineuse du composé comprend un procédé d'administration en deux étapes, et dans une première étape pour l'induction d'une anesthésie générale, le taux de dosage du composé par administration intraveineuse est d'environ 4 mg/heure/kg à environ 30 mg/heure /kg chez le patient et dans une seconde étape pour le maintien de l'anesthésie générale, le taux de dosage du composé par administration intraveineuse est d'environ 0,4 mg/heure/kg à environ 2 mg/heure/kg chez le patient.

6. Composé destiné à être utilisé selon la revendication 3 ou 5, dans lequel l'analgésique narcotique est le rémifentanil et/ou le myorelaxant est le bromure de rocuronium.

7. Composé destiné à être utilisé selon la revendication 1, 5 ou 6, dans lequel le taux d'incidence de l'hypotension en tant qu'effet secondaire est inférieur à 15 %.

8. Composé destiné à être utilisé selon la revendication 2, dans lequel l'utilisation est en combinaison avec un analgésique narcotique et éventuellement un myorelaxant, et dans la première étape, le taux de dosage du composé par administration intraveineuse est d'environ 6 ou 12 mg/heure/kg chez le patient et dans la seconde étape, le taux de dosage du composé par administration intraveineuse est d'environ 0,4 mg/heure/kg à environ 1 mg/heure/kg chez le patient.
